(19) Europäisches Patentamt
European Patent Office
Office européen des brevets

(11) EP 3 977 987 A1

(12) # EUROPEAN PATENT APPLICATION
published in accordance with Art. 153(4) EPC

(43) Date of publication:
**06.04.2022 Bulletin 2022/14**

(21) Application number: **20826953.0**

(22) Date of filing: **12.06.2020**

(51) Int Cl.:
***A61K 9/48*** *(2006.01)* ***A61K 9/00*** *(2006.01)*
***A61K 31/4745*** *(2006.01)* ***A61P 35/00*** *(2006.01)*

(86) International application number:
**PCT/KR2020/007628**

(87) International publication number:
**WO 2020/256349 (24.12.2020 Gazette 2020/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.06.2019 KR 20190073344**

(71) Applicant: **Dae Hwa Pharma. Co., Ltd**
**Hoengseong-gun, Gangwon-do 25228 (KR)**

(72) Inventors:
- **JANG, Jun-Hee**
**Seoul 07988 (KR)**
- **LEE, In-Hyun**
**Gwangju 61260 (KR)**
- **SON, Min-Hee**
**Seongnam-si Gyeonggi-do 13491 (KR)**
- **PARK, Hye-Jin**
**Gwangju-si Gyeonggi-do 12785 (KR)**

(74) Representative: **Turner, Craig Robert et al**
**A.A. Thornton & Co.**
**Octagon Point**
**5 Cheapside**
**London EC2V 6AA (GB)**

(54) **PHARMACEUTICAL COMPOSITION COMPRISING IRINOTECAN FREE BASE FOR ORAL ADMINISTRATION**

(57) The present invention provides a pharmaceutical composition comprising irinotecan free base. The pharmaceutical composition according to the present invention enables the effective oral administration of irinotecan and as such, can increase the convenience of administration of irinotecan and provide a high *in vivo* absorption rate, compared to conventional injections.

**FIG. 1**

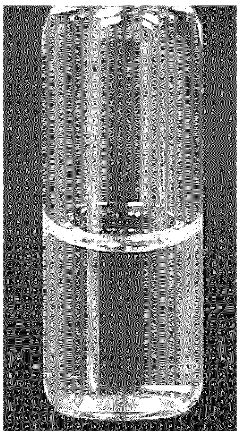

EP 3 977 987 A1

## Description

## TECHNICAL FIELD

The present invention relates to a pharmaceutical composition for oral administration comprising irinotecan free base. More specifically, the present invention relates to a pharmaceutical composition for oral administration comprising irinotecan free base as an active ingredient; and polyoxylglyceride, polyethylene glycol, acyl glycerol, and sorbitan fatty acid ester in specific amounts.

## BACKGROUND ART

Irinotecan may be synthesized from camptothecin and is an anticancer chemotherapeutic agent mainly applied to metastatic colon or rectal cancer. Irinotecan, whose chemical name is (S)-4,11-diethyl-3,4,12,14-tetrahydro-4-hydroxy-3,14-dioxo-1H-pyrano[3',4':6,7]-indolizin o[1,2-b]quinolin-9-yl [1,4'bipiperidine]-1'-carboxylate, has the chemical structure of Formula 1 below.

<Formula 1>

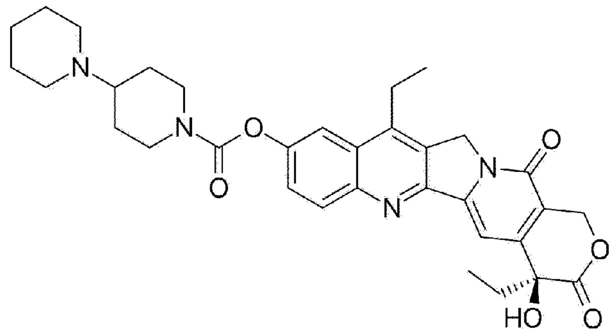

Irinotecan, a topoisomerase I inhibitor, inhibits the action of topoisomerase which plays a role in DNA copying, gene recombination, and transcription. Irinotecan is used for the treatment of cancers, e.g., metastatic rectal cancer or colon cancer. Irinotecan exhibits excellent antitumor activity against a wide variety of experimental tumor models and is being studied specifically for lung cancer, pancreatic cancer, non-Hodgkin's lymphoma, cervical cancer, head and neck cancer, brain tumor, ovarian cancer, etc. (WO 2001/30351). Irinotecan is a prodrug which is metabolized to the active metabolite SN-38 by carboxylesterase in the liver. SN-38 has 100 to 1000 times more potency than irinotecan. Irinotecan and its active metabolite SN-38 bind to the topoisomerase I-DNA complex, thereby preventing DNA unwinding. SN-38, whose chemical name is 7-ethyl-10-hydroxy-camptothecin, has the chemical structure of Formula 2 below.

<Formula 2>

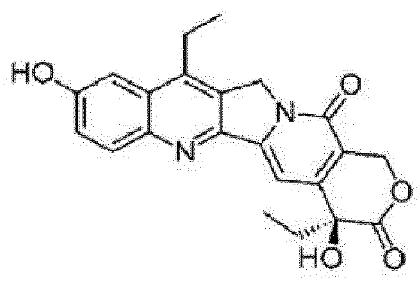

Currently, irinotecan is administered only as an aqueous solution for intravenous infusion over 30 to 90 minutes, weekly or once every three weeks. An aqueous solution of irinotecan hydrochloride trihydrate (CPT-11) for intravenous administration is currently marketed under the trade name CAMPTOSAR®. However, the administration route of the commercially available irinotecan formulation is intravenous injection, which has a disadvantage in that it is necessary to visit a hospital over a long period of time. Solid oral dosage forms, such as tablet formulations, can provide significant

convenience to patients who have to make repeated visits to the clinic or hospital over a long period of time in order to receive intravenous chemotherapy. The development of oral dosage forms significantly improves the quality of life of patients who need to go through multiple treatment cycles, by preventing them from becoming entangled in a hospital visit cycle. In addition, from a pharmacological and economic point of view, it can provide a significant reduction in health care costs if the patient can administer at home. Therefore, an attempt was made to develop an oral formulation that patients can self-administer at home for increasing the convenience of dosing as well as for reducing the costs due to intravenous administration, but a successful oral formulation thereof has not yet been reported.

The efficacy of irinotecan was shown to be dose-dependent and schedule-dependent. It is known that long-term administration of irinotecan at a low dose is more effective and less toxic than short-term administration thereof at a high dose. An effective long-term administration method of irinotecan would be an oral administration. The metabolic ratio of total irinotecan to total SN-38 is higher when administered orally than when administered intravenously. However, a low bioavailability (9%) has been reported upon oral administration of irinotecan; and since irinotecan is a poorly soluble drug, it is difficult to formulate into an oral formulation (EP 2328557).

## DISCLOSURE

### Technical Problem

The present inventors have developed an oral formulation that can solve the problems of the conventional formulation of irinotecan. Especially, the present inventors have found that a pharmaceutical composition for oral administration comprising irinotecan free base as an active ingredient; and polyoxylglyceride, polyethylene glycol, acyl glycerol, and sorbitan fatty acid ester in specific amounts exhibits excellent *in vivo* absorption rate.

Therefore, it is an object of the present invention to provide a pharmaceutical composition for oral administration that can solve the problem of poorly soluble irinotecan free base and improve dosing-convenience and *in vivo* absorption rate.

### Technical Solution

In accordance with an aspect of the present invention, there is provided a pharmaceutical composition for oral administration consisting of: 0.1 ~ 10 % by weight of irinotecan free base; 5 ~ 80 % by weight of polyoxylglyceride; 5 ~ 80 % by weight of polyethylene glycol; 5 ~ 80 % by weight of acyl glycerol; and 5 ~ 25 % by weight of sorbitan fatty acid ester.

The polyoxylglyceride may be one or more selected from the group consisting of caprylocaproyl polyoxylglyceride, lauroyl polyoxylglyceride, linoleoyl polyoxylglyceride, oleoyl polyoxylglyceride, and stearoyl polyoxylglyceride, preferably caprylocaproyl polyoxylglyceride.

The polyethylene glycol may be selected from the group consisting of polyethylene glycol 300, polyethylene glycol 400, polyethylene glycol 600, and polyethylene glycol 900.

The acyl glycerol may be one or more selected from the group consisting of glyceryl behenate, glyceryl oleate, glyceryl stearate, glyceryl palmitostearate, and a complex thereof, preferably an oleoyl glycerol complex having 30 to 65 % by weight of monooleoyl glycerol contents; 15 to 50 % by weight of dioleoyl glycerol contents; and 2 to 20 % by weight of trioleoyl glycerol contents.

The sorbitan fatty acid ester may be one or more selected from the group consisting of sorbitan monostearate, sorbitan diisostearate, sorbitan sesquistearate, sorbitan sesquiisostearate, sorbitan tristearate, sorbitan triisostearate, sorbitan monooleate, sorbitan dioleate, sorbitan sesquioleate, sorbitan trioleate, sorbitan monolaurate, and sorbitan monopalmitate, preferably sorbitan monooleate.

In an embodiment, there is provided a pharmaceutical composition for oral administration in the form of a capsule, which is obtained by filling the pharmaceutical composition for oral administration in a capsule.

## ADVANTAGEOUS EFFECTS

The pharmaceutical composition for oral administration according to the present invention, which comprises irinotecan free base as an active ingredient; and polyoxylglyceride, polyethylene glycol, acyl glycerol, and sorbitan fatty acid ester in specific amounts, can not only solve the problem of poorly soluble irinotecan free base but also improve the bioavailability effectively. Especially, the pharmaceutical composition for oral administration according to the present invention has the advantages of improving the patients' dosing-convenience and reducing the cost due to injection, by making irinotecan which was previously only available as an injection available for oral administration.

## DESCRIPTION OF DRAWINGS

FIG. 1 shows the appearance of the pharmaceutical composition for oral administration, containing irinotecan free base, prepared in Example 1.
FIG. 2 shows the appearance of the formulation prepared in Example 3.
FIG. 3 shows the appearance of the formulation prepared in Comparative Example 1.

## BEST MODE

As used herein, the term "irinotecan free base" refers to a compound in the non-salt form, i.e., a compound in the free base form.

The present invention provides a pharmaceutical composition for oral administration consisting of: 0.1 ~ 10 % by weight of irinotecan free base; 5 ~ 80 % by weight of polyoxylglyceride; 5 ~ 80 % by weight of polyethylene glycol; 5 ~ 80 % by weight of acyl glycerol; and 5 ~ 25 % by weight of sorbitan fatty acid ester.

Irinotecan free base used as an active ingredient in the pharmaceutical composition of the present invention may be used in a therapeutically effective amount. For example, it may be present in an amount ranging from 1 to 30 mg, typically about 15 mg, per ml of unit formulation of the pharmaceutical composition.

The pharmaceutical composition of the present invention comprises irinotecan free base as an active ingredient; and polyoxylglyceride, polyethylene glycol, acyl glycerol, and sorbitan fatty acid ester in specific amounts. In an embodiment of the present invention, there is provided a pharmaceutical composition for oral administration consisting of: 0.1 ~ 10 % by weight of irinotecan free base; 30 ~ 70 % by weight of polyoxylglyceride; 15 ~ 40 % by weight of polyethylene glycol; 5 ~ 20 % by weight of acyl glycerol; and 5 ~ 20 % by weight of sorbitan fatty acid ester.

In the pharmaceutical composition of the present invention, the polyoxylglyceride, which is used as a solubilizing agent, may be one or more selected from the group consisting of caprylocaproyl polyoxylglyceride, lauroyl polyoxylglyceride, linoleoyl polyoxylglyceride, oleoyl polyoxylglyceride, and stearoyl polyoxylglyceride, preferably caprylocaproyl polyoxylglyceride (e.g., Labrasol™).

The polyethylene glycol, which is used as a stabilizing agent, may be selected from the group consisting of polyethylene glycol 300, polyethylene glycol 400, polyethylene glycol 600, and polyethylene glycol 900.

The acyl glycerol, which is used as an emulsifying agent, includes monoacyl glycerol, diacyl glycerol, triacyl glycerol, or a complex thereof, unless otherwise indicated. For example, the acyl glycerol may be one or more selected from the group consisting of glyceryl behenate, glyceryl oleate, glyceryl stearate, glyceryl palmitostearate, and a complex thereof. Preferably, the acyl glycerol may be an oleoyl glycerol complex having 30 to 65 % by weight of monooleoyl glycerol contents; 15 to 50 % by weight of dioleoyl glycerol contents; and 2 to 20 % by weight of trioleoyl glycerol contents. In an embodiment, the oleoyl glycerol complex may be an oleoyl glycerol complex having 32 to 52 % by weight of monooleoyl glycerol contents; 30 to 50 % by weight of dioleoyl glycerol contents; and 5 to 20 % by weight of trioleoyl glycerol contents [e.g., Peceol™ (Gattefosse)]. In another embodiment, the oleoyl glycerol complex may be an oleoyl glycerol complex having 55 to 65 % by weight of monooleoyl glycerol contents; 15 to 35 % by weight of dioleoyl glycerol contents; and 2 to 10 % by weight of trioleoyl glycerol contents [e.g., CAPMUL™ (Abitec)].

The sorbitan fatty acid ester, which is a nonionic surfactant, may be one or more selected from the group consisting of sorbitan monostearate, sorbitan diisostearate, sorbitan sesquistearate, sorbitan sesquiisostearate, sorbitan tristearate, sorbitan triisostearate, sorbitan monooleate, sorbitan dioleate, sorbitan sesquioleate, sorbitan trioleate, sorbitan monolaurate, and sorbitan monopalmitate, preferably sorbitan monooleate (e.g., Span™ 80).

In an embodiment of the present invention, there is provided a pharmaceutical composition for oral administration consisting of: 0.1 ~ 10 % by weight of irinotecan free base; 5 ~ 80 % by weight of caprylocaproyl polyoxylglyceride; 5 ~ 80 % by weight of polyethylene glycol; 5 ~ 80 % by weight of an oleoyl glycerol complex; and 5 ~ 25 % by weight of sorbitan monooleate.

In another embodiment of the present invention, there is provided a pharmaceutical composition for oral administration consisting of: 0.1 ~ 10 % by weight of irinotecan free base; 30 ~ 70 % by weight of caprylocaproyl polyoxylglyceride; 15 ~ 40 % by weight of polyethylene glycol; 5 ~ 20 % by weight of an oleoyl glycerol complex; and 5 ~ 20 % by weight of sorbitan monooleate.

In a particularly preferred embodiment of the present invention, there is provided a pharmaceutical composition for oral administration consisting of: 1.45 % by weight of irinotecan free base; 58.08 % by weight of caprylocaproyl polyoxylglyceride; 21.69 % by weight of polyethylene glycol 300; 9.68 % by weight of an oleoyl glycerol complex; and 9.10 % by weight of sorbitan monooleate.

In another particularly preferred embodiment of the present invention, there is provided a pharmaceutical composition for oral administration consisting of: 1.43 % by weight of irinotecan free base; 34.36 % by weight of caprylocaproyl polyoxylglyceride; 38.48 % by weight of polyethylene glycol 400; 16.18 % by weight of an oleoyl glycerol complex; and 9.55 % by weight of sorbitan monooleate.

Said pharmaceutical composition of the present invention is obtained in the form of a clear oily solution, i.e., in the form of a lipid solution (e.g., see FIG. 1) and may be directly applied to a patient. Therefore, the pharmaceutical composition of the present invention may be formulated as a pharmaceutical composition for oral administration in the form of a lipid solution.

In addition, since the pharmaceutical composition of the present invention is obtained in the form of a clear oily solution (i.e., in the form of a lipid solution), it can be formulated into a capsule form without the formation of a precipitate. Accordingly, the present invention includes a pharmaceutical composition for oral administration in the form of a capsule, which is obtained by filling the pharmaceutical composition for oral administration in the form of a lipid solution in a capsule. The capsule may be a capsule conventionally used in the field of pharmaceutics, preferably soft and hard capsules such as gelatin capsules, pullulan capsules, HPMC capsules, and the like. The pharmaceutical composition for oral administration according to the present invention may be used to treat cancers, including lung cancer, gastric cancer, pancreatic cancer, non-Hodgkin's lymphoma, cervical cancer, head and neck cancer, brain tumor, ovarian cancer, colon cancer, rectal cancer, and the like.

The present invention will be described in further detail with reference to the following examples and experimental examples. These examples and experimental examples are for illustrative purposes only and are not intended to limit the scope of the present invention.

**Example 1: Preparation of a pharmaceutical composition for oral administration comprising irinotecan free base**

A pharmaceutical composition for oral administration comprising irinotecan free base was prepared according to the components and amounts shown in Table 1. The amounts of Table 1 represent the % by weight of each component. Irinotecan free base was completely dissolved in methylene chloride. Polyoxylglyceride (Labrasol™), polyethylene glycol 300, and the oleoyl glycerol complex (Peceol™) were added thereto.

The resulting solution was concentrated under reduced pressure at 40°C to remove methylene chloride and then sorbitan fatty acid ester (Span™ 80) was added thereto.

The resulting solution was stirred at 40°C to prepare a clear oily solution. The appearance thereof is shown in FIG. 1.

Table 1

| L/I | Components | Amount (% by weight) |
|---|---|---|
| Active ingredient | Irinotecan free base | 1.45 |
| Solubilizing agent | Polyoxylglyceride (Labrasol™) | 58.08 |
| Stabilizing agent | Polyethylene glycol 300 | 21.69 |
| Excipient | Sorbitan fatty acid ester (Span™ 80) | 9.68 |
| Emulsifying agent | Oleoyl glycerol complex (Peceol™) | 9.10 |
| Total | | 100.00 |

**Example 2: Preparation of a pharmaceutical composition for oral administration comprising irinotecan free base**

A pharmaceutical composition for oral administration comprising irinotecan free base was prepared according to the components and amounts shown in Table 2. The amounts of Table 2 represent the % by weight of each component. Irinotecan free base was completely dissolved in methylene chloride. Polyoxylglyceride (Labrasol™), polyethylene glycol 400, and the oleoyl glycerol complex (Peceol™) were added thereto.

The resulting solution was concentrated under reduced pressure at 40°C to remove methylene chloride and then sorbitan fatty acid ester (Span™ 80) was added thereto.

The resulting solution was stirred at 40°C to prepare a clear oily solution.

Table 2

| L/I | Components | Amount (% by weight) |
|---|---|---|
| Active ingredient | Irinotecan free base | 1.43 |
| Solubilizing agent | Polyoxylglyceride (Labrasol™) | 34.36 |

(continued)

| L/I | Components | Amount (% by weight) |
|---|---|---|
| Stabilizing agent | Polyethylene glycol 400 | 38.48 |
| Excipient | Sorbitan fatty acid ester (Span™ 80) | 9.55 |
| Emulsifying agent | Oleoyl glycerol complex (Peceol™) | 16.18 |
| Total | | 100.00 |

**Example 3: Preparation of a pharmaceutical composition for oral administration comprising irinotecan free base**

A pharmaceutical composition for oral administration comprising irinotecan free base was prepared according to the components and amounts shown in Table 3. Irinotecan free base was completely dissolved in methylene chloride. Labrasol™, polyethylene glycol 300, and the oleoyl glycerol complex (Peceol™) were added thereto.

The resulting solution was concentrated under reduced pressure at 40°C to remove methylene chloride and then sorbitan fatty acid ester (Span™ 80) was added thereto.

The resulting solution was stirred at 40°C to prepare a clear oily solution. The obtained clear oily solution was filled into a HPMC capsule to prepare a pharmaceutical composition in the form of a capsule. The appearance thereof is shown in FIG. 2.

Table 3

| L/I | Components | Amount per capsule | Amount (% by weight) |
|---|---|---|---|
| Active ingredient | Irinotecan free base | 7.5 mg | 1.45 |
| Solubilizing agent | Polyoxylglyceride (Labrasol™) | 0.3 mL | 58.08 |
| Stabilizing agent | Polyethylene glycol 300 | 0.1 mL | 21.69 |
| Excipient | Sorbitan fatty acid ester (Span™ 80) | 0.05 mL | 9.68 |
| Emulsifying agent | Oleoyl glycerol complex (Peceol™) | 0.05 mL | 9.10 |
| Total | | | 100.00 |

**Experimental Example 1: Measurements of the amounts of irinotecan**

The amounts of irinotecan in the formulations prepared in Examples 1 and 2 were measured. A certain amount of irinotecan standard was taken and dissolved in a mixed solution of acetonitrile, methanol, and acetic acid (100:100:1, v/v/v) to prepare a standard solution. The test solutions were prepared by dissolving the certain amount of each pharmaceutical composition in a mixed solution of acetonitrile, methanol, and acetic acid (100:100:1, v/v/v). The prepared standard solution and test solutions were analyzed with a HPLC under the following conditions; and the amounts of irinotecan were calculated by the following equation.

<HPLC conditions>
HPLC: Shimadzu LC-20AD
Detector: Ultraviolet detector
Wavelength: 255 nm
Column: 4.6 mm×250 mm, 5um, C18
Column temperature: 40 °C
Injection volume: 15 ul

<Equation for calculating the amounts of irinotecan>

$$\text{Amount of rinotecan (\%)} = AT/AS*CS/CT*P$$

AT: Peak area of irinotecan of the test solution
AS: Peak area of irinotecan of the standard solution
CS: Concentration of irinotecan of the standard solution (mg/ml)
CT: Concentration of irinotecan of the test solution (mg/ml)
P: Purity of the irinotecan standard

As the results thereof, the amount of irinotecan in the formulation prepared in Example 1 was confirmed to be 100.4%, and the amount of irinotecan in the formulation prepared in Example 2 was confirmed to be 100.2%.

**Experimental Example 2: Evaluation of *in vivo* absorption rate**

The formulations prepared in Examples 1 and 2 were orally administered to ICR mice (6 weeks old, female), using a gastric sonde at a dose of 225 mg/kg. After the administration, blood samples were collected at 0 minute, 30 minutes, 1 hour, 2 hours, 4 hours, 6 hours, and 8 hours from the mouse by retro-orbital sinus/plexus sampling and then centrifuged at 6000 x g, at 4°C for 20 min. Each supernatant was taken to obtain plasma samples. A mixture of acetonitrile and 1 mM phosphoric acid (9:1, v/v) (300 μL) was added to the obtained plasma samples (100 μL). Each resulting sample was stirred on the vortex mixer and then centrifuged at 14,000 x g at 4°C for 20 minutes. Each supernatant (200 μL) was taken and then 600 μL of 0.1 M potassium phosphate (pH 4.2) was added thereto. The concentrations of irinotecan and SN-38 in the blood samples were analyzed, using an HPLC-FLD detector.
The *in vivo* absorption patterns of the formulations prepared in Examples 1 and 2 in ICR mice were evaluated, using a HPLC under the following conditions.

<HPLC conditions>
HPLC: Agilent 1260
Detector: Fluorescence detector
Wavelength: 228(excitation)-543(emission) nm for 20 min(SN-38)
Column: 4.6 mm×250 mm, 5um, C18
Column temperature: 30 °C
Injection volume: 30 ul

The pharmacokinetic parameters for irinotecan and its active metabolite (SN-38) were shown in Table 4 below.

Table 4

| | Material for analysis | Cmax (ng/ml) | Tmax (h) | $AUC_{0-8h}$ (ng·h/ml) | $AUC_{0-inf}$ (ng·h/ml) |
|---|---|---|---|---|---|
| Example 1 | Irinotecan | 2175.18 | 1 | 8237.02 | 10930.32 |
| | SN-38 | 319.05 | 0.5 | 1367.98 | 3224.13 |
| Example 2 | Irinotecan | 2371.07 | 1 | 9053.62 | 12121.42 |
| | SN-38 | 361.95 | 0.5 | 1563.20 | 3718.80 |

As shown in Table 4, it can be seen that the pharmaceutical compositions obtained according to the present invention are rapidly absorbed with a Tmax of 1 hour and exhibits high bioavailability.

**Comparative Examples**

Formulations comprising irinotecan free base were prepared according to the components and amounts shown in Table 5. The amounts of Table 5 represent the % by weight of each component. Irinotecan free base was completely dissolved in methylene chloride. Any one of polyoxylglyceride (Labrasol™), polyethylene glycol 300, polyethylene glycol 400, oleoyl glycerol complex (Peceol™), and sorbitan fatty acid ester (Span™ 80) was added thereto. The resulting solutions were concentrated under reduced pressure at 40°C for removing methylene chloride, so as to prepare the formulations of Comparative Examples 1 to 5. All of the formulations of Comparative Examples 1 to 5 were in the form of an opaque solution having precipitations. FIG. 3 shows the appearance of the formulation of Comparative Example 1.

Table 5

| L/I | Components | Comparative Example (% by weight) | | | | |
|---|---|---|---|---|---|---|
| | | 1 | 2 | 3 | 4 | 5 |
| Active ingredient | Irinotecan free base | 1.48 | 1.48 | 1.48 | 1.48 | 1.48 |
| Solubilizing agent | Polyoxylglyceride (Labrasol™) | 98.52 | - | - | - | - |
| Stabilizing agent Additive | Polyethylene glycol 300 | - | 98.52 | - | - | - |
| | Polyethylene glycol 400 | - | - | 98.52 | - | - |
| Excipient | Sorbitan fatty acid ester (Span™ 80) | - | - | - | 98.52 | - |
| Emulsifying agent | Oleoyl glycerol complex (Peceol™) | - | - | - | - | 98.52 |
| Total | | 100.00 | 100.00 | 100.00 | 100.00 | 100.00 |

**Claims**

1. A pharmaceutical composition for oral administration consisting of:

   0.1 ~ 10 % by weight of irinotecan free base;
   5 ~ 80 % by weight of polyoxylglyceride;
   5 ~ 80 % by weight of polyethylene glycol;
   5 ~ 80 % by weight of acyl glycerol; and
   5 ~ 25 % by weight of sorbitan fatty acid ester.

2. The pharmaceutical composition according to claim 1, consisting of:

   0.1 ~ 10 % by weight of irinotecan free base;
   30 ~ 70 % by weight of polyoxylglyceride;
   15 ~ 40 % by weight of polyethylene glycol;
   5 ~ 20 % by weight of acyl glycerol; and
   5 ~ 20 % by weight of sorbitan fatty acid ester.

3. The pharmaceutical composition according to claim 1, wherein the polyoxylglyceride is one or more selected from the group consisting of caprylocaproyl polyoxylglyceride, lauroyl polyoxylglyceride, linoleoyl polyoxylglyceride, oleoyl polyoxylglyceride, and stearoyl polyoxylglyceride.

4. The pharmaceutical composition according to claim 3, wherein the polyoxylglyceride is caprylocaproyl polyoxylglyceride.

5. The pharmaceutical composition according to claim 1, wherein the polyethylene glycol is selected from the group consisting of polyethylene glycol 300, polyethylene glycol 400, polyethylene glycol 600, and polyethylene glycol 900.

6. The pharmaceutical composition according to claim 1, wherein the acyl glycerol is one or more selected from the group consisting of glyceryl behenate, glyceryl oleate, glyceryl stearate, glyceryl palmitostearate, and a complex thereof.

7. The pharmaceutical composition according to claim 6, wherein the acyl glycerol is an oleoyl glycerol complex having 30 to 65 % by weight of monooleoyl glycerol contents; 15 to 50 % by weight of dioleoyl glycerol contents; and 2 to 20 % by weight of trioleoyl glycerol contents.

8. The pharmaceutical composition according to claim 1, wherein the sorbitan fatty acid ester is one or more selected from the group consisting of sorbitan monostearate, sorbitan diisostearate, sorbitan sesquistearate, sorbitan sesquiisostearate, sorbitan tristearate, sorbitan triisostearate, sorbitan monooleate, sorbitan dioleate, sorbitan sesquioleate, sorbitan trioleate, sorbitan monolaurate, and sorbitan monopalmitate.

**9.** The pharmaceutical composition according to claim 8, wherein the sorbitan fatty acid ester is sorbitan monooleate.

**10.** The pharmaceutical composition according to claim 1, consisting of:

0.1 ~ 10 % by weight of irinotecan free base;
5 ~ 80 % by weight of caprylocaproyl polyoxylglyceride;
5 ~ 80 % by weight of polyethylene glycol;
5 ~ 80 % by weight of an oleoyl glycerol complex; and
5 ~ 25 % by weight of sorbitan monooleate.

**11.** The pharmaceutical composition according to claim 1, consisting of:

0.1 ~ 10 % by weight of irinotecan free base;
30 ~ 70 % by weight of caprylocaproyl polyoxylglyceride;
15 ~ 40 % by weight of polyethylene glycol;
5 ~ 20 % by weight of an oleoyl glycerol complex; and
5 ~ 20 % by weight of sorbitan monooleate.

**12.** The pharmaceutical composition according to claim 1, consisting of:

1.45 % by weight of irinotecan free base;
58.08 % by weight of caprylocaproyl polyoxylglyceride;
21.69 % by weight of polyethylene glycol 300;
9.68 % by weight of an oleoyl glycerol complex; and
9.10 % by weight of sorbitan monooleate.

**13.** The pharmaceutical composition according to claim 1, consisting of:

1.43 % by weight of irinotecan free base;
34.36 % by weight of caprylocaproyl polyoxylglyceride;
38.48 % by weight of polyethylene glycol 400;
16.18 % by weight of an oleoyl glycerol complex; and
9.55 % by weight of sorbitan monooleate.

**14.** A pharmaceutical composition for oral administration in the form of a capsule, which is obtained by filling the pharmaceutical composition for oral administration according to any one of the claims 1 to 13 in a capsule.

FIG. 1

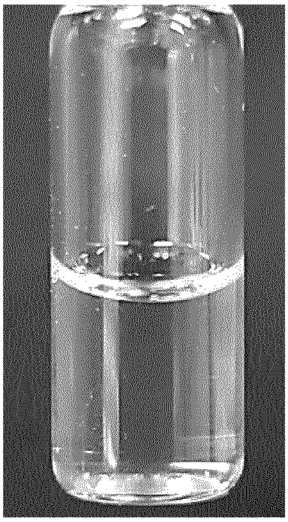

FIG. 2

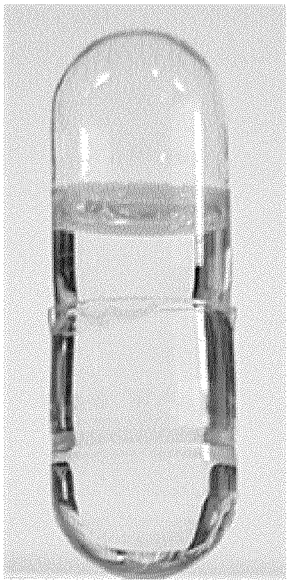

FIG. 3

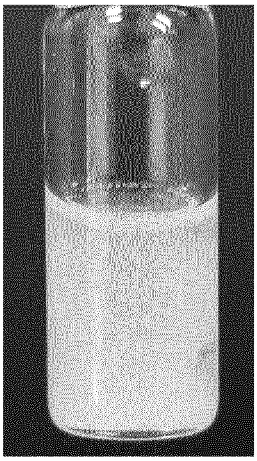

**INTERNATIONAL SEARCH REPORT**

International application No. **PCT/KR2020/007628**

**A. CLASSIFICATION OF SUBJECT MATTER**

***A61K 9/48***(2006.01)i; ***A61K 9/00***(2006.01)i; ***A61K 31/4745***(2006.01)i; ***A61P 35/00***(2006.01)i

According to International Patent Classification (IPC) or to both national classification and IPC

**B. FIELDS SEARCHED**

Minimum documentation searched (classification system followed by classification symbols)

A61K 9/48; A61K 31/381; A61K 31/4741; A61K 31/4745; A61K 31/704; A61K 47/10; A61K 47/12; A61K 47/14; A61K 9/16; A61K 9/00; A61P 35/00

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 이리노테칸 (irinotecan), 경구투여제 (oral composition), 폴리옥실글리세라이드 (polyoxyl glyceride), 폴리에틸렌글리콜(polyethylene glycol), 아실글리세롤 (acylglycerol), 솔비탄 지방산 에스테르 (sorbitan fatty acid ester)

**C. DOCUMENTS CONSIDERED TO BE RELEVANT**

| Category* | Citation of document, with indication, where appropriate, of the relevant passages | Relevant to claim No. |
|---|---|---|
| A | KR 10-2016-0107329 A (ONCORAL PHARMA APS) 13 September 2016. See abstract; paragraphs [0054], [0061] and [0076]; and claims 1-49. | 1-14 |
| A | YOSHINO, K. et al. Comparative studies of irinotecan-loaded polyethylene glycol-modified liposomes prepared using different PEG-modification methods. Biochimica et Biophysica Acta (BBA)-Biomembranes. 2012, vol. 1818, no. 11, pp. 2901-2907. See abstract. | 1-14 |
| A | KR 10-2016-0093611 A (ALRISE BIOSYSTEMS GMBH) 08 August 2016. See abstract; and claims 1-19. | 1-14 |
| A | KR 10-2017-0003143 A (HANMI PHARM. CO., LTD.) 09 January 2017. See abstract; and claims 1-14. | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 November 2020** | **09 November 2020** |

| Name and mailing address of the ISA/KR | Authorized officer |
|---|---|
| **Korean Intellectual Property Office**<br>**Government Complex Daejeon Building 4, 189, Cheongsa-ro, Seo-gu, Daejeon, Republic of Korea**<br>**35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2020/007628**

**C. DOCUMENTS CONSIDERED TO BE RELEVANT**

| Category* | Citation of document, with indication, where appropriate, of the relevant passages | Relevant to claim No. |
|---|---|---|
| A | KR 10-2005-0116166 A (PHARMACIA AND UPJOHN COMPANY LLC) 09 December 2005. See abstract; and claims 1-26. | 1-14 |
| PX | KR 10-2019-0076585 A (DAE HWA PHARMA. CO., LTD.) 02 July 2019. See abstract; and claims 1-12. | 1-14 |

Form PCT/ISA/210 (second sheet) (July 2019)

**INTERNATIONAL SEARCH REPORT**
**Information on patent family members**

International application No.
**PCT/KR2020/007628**

| Patent document cited in search report | Publication date (day/month/year) | Patent family member(s) | Publication date (day/month/year) |
|---|---|---|---|
| KR 10-2016-0107329 A | 13 September 2016 | CA 2973906 A1 | 23 July 2015 |
| | | CN 106103451 A | 09 November 2016 |
| | | CN 106103451 B | 29 October 2019 |
| | | CN 110946836 A | 03 April 2020 |
| | | EP 3094635 A1 | 23 November 2016 |
| | | EP 3094635 B1 | 04 July 2018 |
| | | ES 2680444 T3 | 07 September 2018 |
| | | JP 2017-502979 A | 26 January 2017 |
| | | JP 6704345 B2 | 03 June 2020 |
| | | US 10143657 B2 | 04 December 2018 |
| | | US 2016-0331692 A1 | 17 November 2016 |
| | | US 2018-0369153 A1 | 27 December 2018 |
| | | WO 2015-107131 A1 | 23 July 2015 |
| KR 10-2016-0093611 A | 08 August 2016 | CA 2928772 A1 | 11 June 2015 |
| | | EP 3076951 A1 | 12 October 2016 |
| | | EP 3076951 B1 | 30 September 2020 |
| | | JP 2016-539953 A | 22 December 2016 |
| | | JP 6505705 B2 | 24 April 2019 |
| | | US 2016-0303102 A1 | 20 October 2016 |
| | | WO 2015-082562 A1 | 11 June 2015 |
| KR 10-2017-0003143 A | 09 January 2017 | AR 105203 A1 | 13 September 2017 |
| | | AU 2016-286804 A1 | 30 November 2017 |
| | | BR 112017028468 A2 | 28 August 2018 |
| | | CA 2988079 A1 | 05 January 2017 |
| | | CL 2017003437 A1 | 22 June 2018 |
| | | CN 107949375 A | 20 April 2018 |
| | | CO 2017013317 A2 | 28 March 2018 |
| | | CR 20170604 A | 27 April 2018 |
| | | DO P2017000307 A | 15 March 2018 |
| | | EC SP17085443 A | 31 January 2018 |
| | | EP 3300483 A1 | 04 April 2018 |
| | | GT 201700280 A | 10 October 2019 |
| | | HK 1246656 A1 | 14 September 2018 |
| | | JP 2018-519279 A | 19 July 2018 |
| | | MX 2017014994 A | 15 August 2018 |
| | | PE 20181040 A1 | 03 July 2018 |
| | | PH 12017502376 A1 | 25 June 2018 |
| | | RU 2017143849 A | 01 August 2019 |
| | | RU 2017143849 A3 | 25 September 2019 |
| | | RU 2716595 C2 | 13 March 2020 |
| | | SG 10201913274 A | 30 March 2020 |
| | | SV 2017005603 A | 12 March 2018 |
| | | TW 201713339 A | 16 April 2017 |
| | | US 2018-0153878 A1 | 07 June 2018 |
| | | UY 36761 A | 30 December 2016 |
| | | WO 2017-003120 A1 | 05 January 2017 |
| | | ZA 201708429 B | 26 June 2019 |
| KR 10-2005-0116166 A | 09 December 2005 | AU 2004-233743 A1 | 11 November 2004 |
| | | BR PI0409870 A | 16 May 2006 |
| | | CA 2523152 A1 | 11 November 2004 |

Form PCT/ISA/210 (patent family annex) (July 2019)

**INTERNATIONAL SEARCH REPORT**
**Information on patent family members**

International application No.
**PCT/KR2020/007628**

| Patent document cited in search report | Publication date (day/month/year) | Patent family member(s) | Publication date (day/month/year) |
|---|---|---|---|
| | | CL 2004000888 A1 | 18 March 2005 |
| | | CN 1774249 A | 17 May 2006 |
| | | EP 1620099 A1 | 01 February 2006 |
| | | JP 2006-524678 A | 02 November 2006 |
| | | MX PA05011568 A | 14 December 2005 |
| | | TW 200509925 A | 16 March 2005 |
| | | US 2004-0266704 A1 | 30 December 2004 |
| | | WO 2004-096223 A1 | 11 November 2004 |
| | | ZA 200508696 B | 26 July 2006 |
| KR 10-2019-0076585 A | 02 July 2019 | CN 111511349 A | 07 August 2020 |
| | | KR 10-2066402 B1 | 15 January 2020 |
| | | WO 2019-124789 A1 | 27 June 2019 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200130351 A
- EP 2328557 A